# EUROPÄISCHE PATENTSCHRIFT

(11) **EP 2 432 543 B1**
(45) Veröffentlichungstag und Bekanntmachung des Hinweises auf die Patenterteilung: **17.12.2014**
(21) Anmeldenummer: 10722547.6
(22) Anmeldetag: 16.04.2010
(51) Int. Cl.: A61M 27/00, A61M 1/00

(54) **FLÄCHENDRAINAGE ZUR ABLEITUNG VON WUNDSEKRET VON GROßFLÄCHIGEN WUNDEN UND AUS KÖRPERHÖHLEN**
AREAL DRAINAGE FOR DRAINING WOUND SECRETION FROM LARGE-SURFACE-AREA WOUNDS AND FROM BODY CAVITIES
DRAINAGE SURFACIQUE POUR ÉVACUER UNE SÉCRÉTION PROVENANT DE LARGES PLAIES ET DE CAVITÉS CORPORELLES

(30) Priorität: 20.04.2009 DE 102009017960
(43) Veröffentlichungstag der Anmeldung: 28.03.2012
(73) Patentinhaber: WESIL GbR, 37327 Birkungen (DE)
(72) Erfinder: NEUBAUER, Norbert, 38820 Halberstadt (DE); IHLE, Peter, 37327 Leinefelde (DE); WEISE, Steffen, 99755 Ellrich (DE)
(74) Vertreter: Carlsohn, Alexander
(86) Internationale Anmeldenummer: PCT/DE2010/000443
(87) Internationale Veröffentlichungsnummer: WO 2010/121593

(56) Entgegenhaltungen:
- WO-A1-2008/131895
- DE-A1- 3 524 552
- US-A- 3 860 008
- US-A1- 2004 176 745
- US-A1- 2005 065 484

## Beschreibung

Die Erfindung betrifft eine Flächendrainage, bevorzugt zur Ableitung des Sekretflusses aus großflächigen Wunden und Körperhöhlen.

Nach dem bekannten Stand der Technik existiert eine Vielzahl von Vorschlägen zum Anwendungsgebiet vorliegender Erfindung, von denen einige typische nachfolgend beispielhaft aufgeführt werden sollen:

So ist in DE 88 13 995 eine chirurgische Drainage zur Ableitung von Wundsekret aus menschlichen oder tierischen Körperhöhlen nach operativen Eingriffen beschrieben, die sich eines dünnwandigen Flachprofils mit innen, in Längsrichtung verlaufenden Rippen bedient, die ein Kollabieren der Flachdrainage verhindern sollen und an welches ein Schlauch mit Rundprofil ohne Innenrippen anvulkanisiert ist. Bereits in dieser Schrift wird mitgeteilt, dass der Einsatz flach profilierter Drainagen mit innerhalb der Drainagen vorgesehenen Rippen zur Unterdrückung des Kollabierens der Drainage seit Jahren bekannt ist.

Im Rahmen der bekannten Redon-Technik, bei der ein Unterduck an die Absaugdrainage angelegt ist, ist aus DE 33 25 920 C2 ein Drainageschlauch bekannt, bei dem ein Grundschlauch, der mit vier außenseitigen, rinnenartigen Vertiefungen, in die Perforationslöcher eingebracht sind, versehen ist, wobei die Vertiefungen und der perforierte Teil mit einem eng anliegenden, dünnwandigen, perforierten Überzugschlauch überzogen sind. Das Sekret soll nach diesem Vorschlag also zuerst durch die Perforation des Überzugschlauches und dann durch die Perforation der Vertiefungen des Grundschlauches fließen, um der Gefahr des Hineinsaugens von Körpergewebe in die Perforationslöcher zu begegnen.

In der DE 198 05 096 C2 wird ein Kapillar-Drainageschlauch-System beschrieben, bei dem ein Drainageschlauch jeweils an seiner Außen- und Innenwandung mit einem Kapillarprofil versehen ist, welches ebenfalls ein Kollabieren des Schlauchs bei Unterdruck vermeiden und einen besseren Sekretabfluss ermöglichen soll.

In der DE 20 2008 004 737 U1 wird ein Labyrinth-Drainageschlauch-System beschrieben, bei dem der Drainageschlauch einstrangig oder mehrstrangig, als Drainageschwanz mit einem Labyrinth versehen, mit einem Ableitungsschlauch stoffschlüssig verbunden ist und durch die Ankopplung eines Ankopplungsstückes ein Flächenlabyrinth ermöglicht wird.

Eine vergleichbare Lösung einer Flächendrainage ist in WO 2008/131895 A1 beschrieben, bei welcher zwischen zwei flexiblen Folien ein Labyrinth zur Sekretabführung vorgesehen ist, wobei der Sekreteintritt in die zuschneidbare Drainage seitlich und über in den flexiblen Folien vorgesehene Perforationen erfolgt, wobei sich an die vorgesehene Drainage ein Ableitungsschlauch anschließt.

Der Nachteil der vorstehend beispielhaft aufgeführten Lösungen besteht hauptsächlich darin, dass sämtliche Schläuche beziehungsweise Schlauchteile, die in der Wunde platziert sind, perforiert oder geschlitzt ausgeführt werden müssen und das Sekret erst einen Weg zum entsprechenden Perforationsloch finden muss, ehe es einer Ableitung zuführbar ist.

Durch die geringe Öffnungsfläche der Perforationen kann das Körpergewebe die Perforationslöcher jedoch sehr schnell umhüllen, so dass ein effektives Ableiten des Sekretes recht schnell verhindert wird, da sich um den Drain ein Hohlraum bildet, der nur sehr begrenzt das Wundsekret ableiten kann, wodurch häufigere, den Patienten belastende Drainwechsel erforderlich werden.

Bei bekannten Flächendrainagen, die grundsätzlich zwar eine effektivere Sekretabsaugung erlauben, entsteht zusätzlich der Nachteil, dass ihre Entnahme, bei erforderlichen Drainagewechseln, durch geringe äußere Körperöffnungen mit Problemen verbunden ist und vom Patienten schmerzhaft wahrgenommen wird. US 2005/0065484 offenbart eine Flächendrainage mit bioabsorbierbarem Material.

Es ist daher die Aufgabe vorliegender Erfindung, eine Flächendrainage anzugeben, die einen guten Sekretabfluss über längere Zeiträume gewährleistet, nicht zur Umhüllung oder Verwachsung mit dem Körpergewebe neigt und die unkompliziert und schmerzfrei aus der Wunde entfernt werden kann. Die vorliegende Erfindung soll dabei eine große Drainagefläche schaffen, bei der keine Röhre um den Drain entsteht, wie nach dem bekannten Stand der Technik üblich, das Sekret ungestört in die Drainagefläche fließt und erst dann durch einen Ableitungsschlauch in ein Sammelbehältnis abgeleitet wird.

Der Erfindung liegt dabei die Entdeckung zugrunde, dass entgegen aller Erwartungen, rein flächig ausgedehnte Körper weit weniger zu Verwachsungen mit dem Körpergewebe neigen, als bekannte Flächendrainagen, die mit Perforationen versehen sind.

Die Aufgabe der Erfindung wird durch die kennzeichnenden Merkmale des Patentanspruchs 1 gelöst. Vorteilhafte Ausgestaltungen sind Gegenstand der nachgeordneten Ansprüche.

Das Wesen vorliegender Erfindung besteht darin, dass eine Flachdrainage zur Ableitung des Sekretflusses aus großflächigen Wunden und Körperhöhlen ein Drainagemittel und einen Abflussschlauch umfasst, wobei das Drainagemittel gebildet ist durch wenigstens eine biokompatible, dünne flexible und reißfeste Folie, die von wenigstens zwei, sich über die Folienlänge erstreckende und mit ihr verbundene, vorzugsweise elastische Stege erfasst ist, wobei die Stege einseitig mit dem Abflussschlauch, respektive einem schlauchförmigen Zwischenstück, in eine zugfeste Verbindung gebracht sind und die Folie im Bereich des der Wunde zugewandten Endes des Abflussschlauchs von diesem erfasst ist.

Die im Rahmen der Erfindung zum Einsatz gelangenden Folien mit antiadhäsiver Oberfläche, bestehen bevorzugt aus Polyethylen (PE), Polyurethan (PUR), Polypropylen (PP) oder Silikon und können als Dreieckfläche, Kreisfläche, Rechteckfläche usw. beliebig ausgebildet und/oder zuschneidbar sein. Diese Art der Folien sind für andere medizinische Anwendungen an sich bereits bekannt und bedürfen deshalb hier keiner weiteren Ausführungen. Auf dem Gebiet der Vakuumwundbehandlung werden verlegte, anders ausgebildete Drainagemittel und weiteres medizinisches Instrumentarium nach außen mit solchen Folien überspannt, um ein angelegtes Vakuum aufrecht zu erhalten. Ebenfalls sind vergleichbare Folien aus dem Bereich der Narbenbehandlung bekannt. All diese Folien, mit Zulassungen für die Medizin, können also im Rahmen vorliegender Erfindung ohne Beschränkung Verwendung finden, so sie nur hinreichend dünn, reißfest und flexibel sind.

Erfindungswesentlich ist ihre Verwendung als Drainagemittel und ihre Art der Anbringung an einen Abflussschlauch, wie im Folgenden anhand mehrerer Ausführungsbeispiele detaillierter beschrieben wird.

Die die Ausführungsbeispiele näher erläuternden Figuren zeigen schematisch:
- Fig. 1: eine erste mögliche Ausführungsform in Draufsicht mit allen zur Gesamtdrainage gehörenden möglichen Komponenten;
- Fig. 1a: einen Schnitt allein durch die Flächendrainage entlang einer Schnittebene A-A nach Fig. 1;
- Fig. 2: eine zweite Ausführungsmöglichkeit einer Stegausbildung;
- Fig. 3: eine dritte Ausführungsmöglichkeit einer Stegausbildung;
- Fig. 4: eine weitere Ausbildungsmöglichkeit der Flächendrainage als Folienstrang;
- Fig. 5: eine mögliche erste Anbindungsart der Flächendrainage an einen Abflussschlauch;
- Fig. 6a: und b zwei Varianten einer zweiten möglichen Anbindungsart der Flächendrainage an einen Abflussschlauch und
- Fig. 7: eine dritte mögliche Anbindungsart der Flächendrainage an einen Abflussschlauch mit Perforationen unter zusätzlichem Einsatz einer beispielhaften Einwachsschutzvorrichtung.

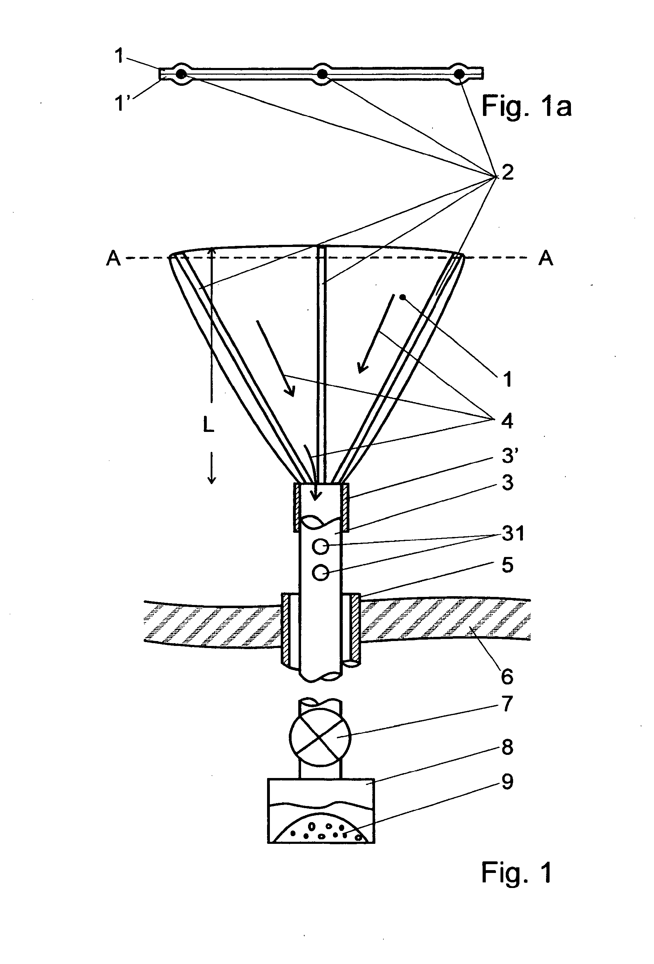

Figur 1 zeigt eine erste mögliche Ausführungsform einer Flächendrainage in Draufsicht. Im Beispiel ist eine Flächenfolie 1, hier im Wesentlichen in dreieckiger Form, vorgesehen. Ohne Beschränkung der Erfindung liegen bevorzugte Abmaße der Dreiecksfläche bei 130 mm x 100 mm x 60 mm. Diese Folie ist mit erfindungsgemäßen Stegen 2 stoff- und/oder formschlüssig verbunden. Dabei sind im Rahmen der Erfindung wenigstens zwei äußere Stege 2 erforderlich, die im Wesentlichen der äußeren seitlichen Folienberandung in Geometrie und Länge L folgen. Im speziellen Beispiel nach Fig. 1 ist zusätzlich mittig ein solcher Steg 2, wie dargestellt, vorgesehen. Genannte Stege 2 sind dabei einseitig mit dem Abflussschlauch 3 direkt oder indirekt über ein Zwischenstück 3' in eine zugfeste Verbindung gebracht, wobei die Folie 1 im Bereich des der Wunde zugewandten Endes des Abflussschlauchs 3 von diesem oder genanntem Zwischenstück 3' erfasst ist. Die Funktionen, die genanntes Zwischenstück 3' im Rahmen der Erfindung übernehmen soll, sind die gleichen, die auch direkt der Endbereich des Abflussschlauches übernimmt. Es kann herstellungstechnisch jedoch vorteilhaft sein, das Zwischenstück 3' vorzusehen, welches dann zugfest mit dem Abflussschlauch zu verbinden ist, wenn, worauf später eingegangen wird, die Stege und das Zwischenstück als einstückiges Teil gefertigt werden sollen. Auf die spezielle Folienanbindung an die Stege 2 wird weiter unten näher eingegangen.

Das dargestellte Beispiel soll die bereits verlegte erfindungsgemäße Flachdrainage in einer Körperhöhle zeigen und zur Vervollständigung sollen die, teilweise außerhalb vorliegender Erfindung liegenden, weiter dargestellten Baugruppen benannt werden. An einen an sich üblichen Abflussschlauch 3, der zusätzliche an sich ebenfalls übliche Perforationen 31 aufweisen kann, schließen sich bspw. eine Vakuumquelle 7 und eine Sekretauffangbehälter 8, der vorzugsweise mit einer das Wundsekret bindenden Geliermasse 9 bestückt ist, an. All diese zusätzlichen Baugruppen beschränken vorliegende Erfindung jedoch nicht. So kann die erfindungsgemäße Flachdrainage auch als reine Passivdrainage, d.h. ohne Vakuumabsaugung, betrieben werden, was in der Hand des Mediziners liegt. Ein großer Vorteil der Erfindung zeigt sich jedoch in der Art der Platzierungsmöglichkeit der Flachdrainage an sich. So ist aus Fig. 1 schematisch ersichtlich, das die Verlegung der Flachdrainage durch einen Einstich durch die Haut 6 erfolgte und die gesamte Flachdrainage über eine teilweise dargestellte Einführhülse 5 vorgenommen wurde, deren Innendurchmesser so bemessen ist, dass sie die Aufnahme des Abflussschlauches 3, respektive des mit ihm verbundenen Zwischenstücks 3', und der zusammengerollten oder gefalteten Flachdrainage, bestehend aus der Folie 1 und den Stegen 2, ermöglicht. Die Ausbildung der Flachdrainage an sich ist also derart, dass sie auch im Rahmen minimalinvasiver Operationsmethoden zum Einsatz gelangen kann.

Der Außendurchmesser der eingesetzten Einführhülse 5 kann somit in der Größenordnung von 12 mm festgelegt sein. Bei der Verlegung der Flachdrainage wird die Einführungshülse 5 über die Flächenfolie 1 geschoben, die sich dabei im Inneren der Hülse 5 einbettet (nicht dargestellt). Danach erfolgt die Platzierung durch einen Einstich in der Haut 6 und einem Einschieben der Flachdrainage in den Wundbereich. Nach der Platzierung wird die Einführungshülse 5 zweckmäßiger Weise entfernt und entsorgt.

Durch Ausrichtung der Stege 2 im Wundbereich, bspw. wie in Fig. 1 dargestellt, spreizt sich die Folie 1 auf und bedingt einen äußerst effektiven ganzflächigen Abfluss des Wundsekretes, wie in ersten operativen Versuchen gefunden wurde.

Das vorgeschlagene Drainagesystem ermöglicht einen optimalen gefahrfreien Sekretfluss von einer großen Fläche, ein ungewolltes Verkleben wird überraschend verhindert, Behinderungen des Sekretabflusses werden vermieden und das System kann, bedingt durch seinen erfindungsgemäßen Aufbau, leicht und sicher entfernt werden. Die Liegezeiten unter gleichzeitiger voller Funktionsfähigkeit der Drainage sind, gegenüber eingangs zitierter Flachdrainagen nach dem Stand der Technik, hier erheblich verlängert.

Pfeile 4 bezeichnen in Fig. 1 beispielhaft den Weg des Wundsekretflusses auf der Folienfläche in Richtung des Abflussschlauches 3. Die vorgesehenen Stege 2 übernehmen neben der Stabilisierung und flächenmäßigen Aufspannung der Folie 1 zusätzlich bevorzugte Leitwegfunktionen für den Sekretfluss, der letztendlich im Abflussschlauch 3 mündet.

Letztere Funktion wird deutlicher, wenn man sich das Schnittbeispiel entlang einer Ebene A-A betrachtet, welches für eine Ausführungsmöglichkeit in Fig. 1a beispielhaft dargestellt ist. Die erfindungsgemäße Flachdrainage lässt sich besonders leicht, unter Einsatz kommerziell verfügbarer Komponenten aufbauen, wenn die Stege 2 zwischen zwei Folien eingebettet werden. Die Folien 1 und 1' können dann mit den Stegen 2 bspw. verklebt oder thermisch verbunden werden. Andere, weiter unten beschriebene Ausbildungen liegen aber ebenfalls im Rahmen dieser Erfindung.

Für das zum Einsatz gelangende medizinisch erprobte Folienmaterial kommt hier insbesondere eine Polyurethanfolie in Betracht, die selbst bei Dicken in der anzustrebenden Größenordnung von 0,08 mm eine hinreichend hohe Zerreißfestigkeit aufweist, was für die Platzierung im Wundbereich und bei der Entnahme der gesamten Flächendrainage aus der Wunde wesentlich ist.

Die Stege 2 sollen im Rahmen der Erfindung zumindest elastisch ausgebildet sein. Dabei können sie in sich starr ausgeführt sein oder aber auch mit einer gewissen Vorspannung an den Abflussschlauch 3, respektive das Zwischenstück 3', derart angebunden sein, dass eine regenschirmartige selbsttätige Aufspannung der Folie im verlegten Zustand erfolgt. Die Stege 2 können aber auch als Angriffpunkte für den Operateur zur Ausrichtung und Platzierung der Flächendrainage im Wundbereich dienen.

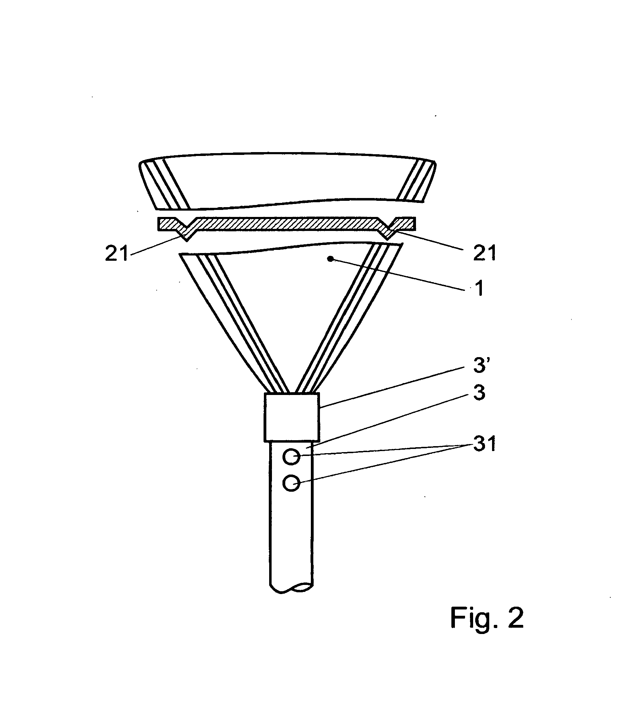

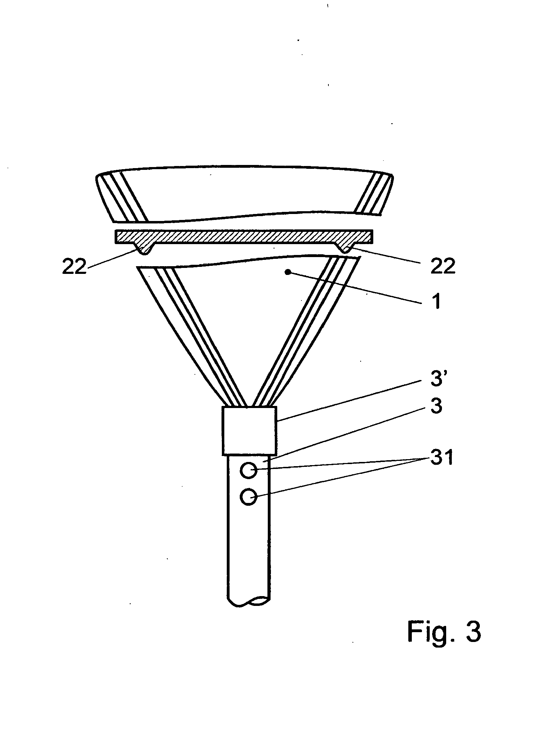

Ebenso liegt es im Rahmen der Erfindung, die Stege 2 als profilierte Ausbuchtungen 21 oder Verdickungen 22 auszuführen, wie es beispielhaft in Fig. 2 oder Fig. 3 in einer teilgeschnittenen Darstellung veranschaulicht ist.

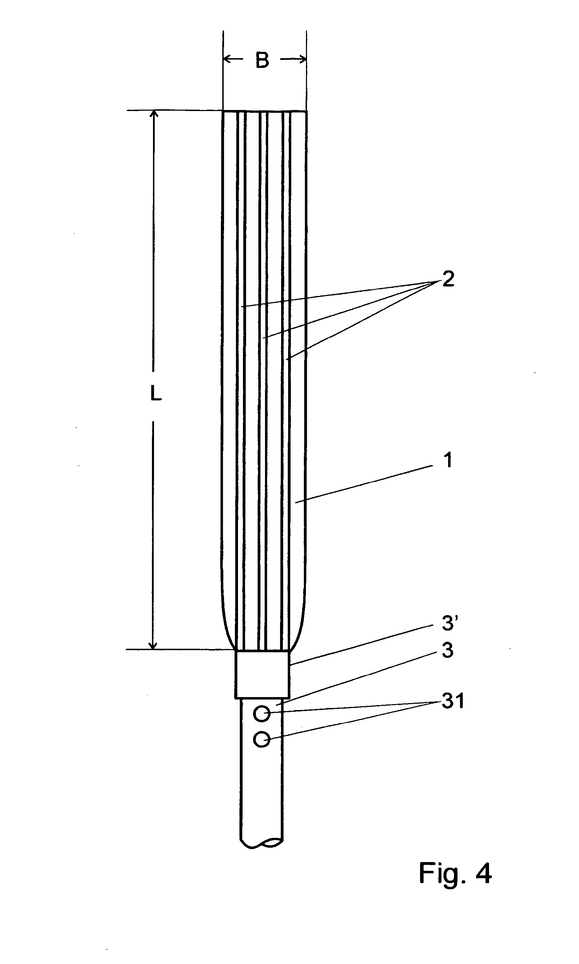

Auch die Ausbildung der Flächendrainage als Folienstrang, wie in Fig. 4 dargestellt, liegt im Rahmen der Erfindung und kann bei entsprechender Indikation angeraten sein. Wie in Fig. 4 schematisch dargestellt, verlaufen in einer solchen Ausführung die Stege 2 parallel zueinander und die Länge L übersteigt die Breite B der Folie 1 um ein Mehrfaches. Die Art der Stegausbildung kann dabei jeweils nach einer der vorstehend beschriebenen Möglichkeiten erfolgen. Die hier vorgesehene parallele Führung der Stege 2 bietet technologische Vorteile bei der Herstellung, da die einzelnen Flachdrainagen hier als fortlaufende Meterware vorkonfektioniert werden können.

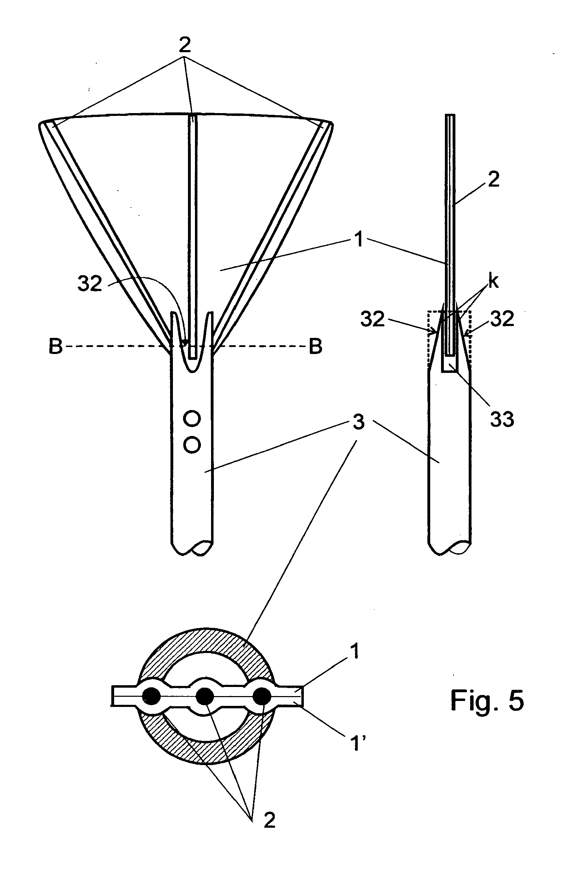

In Figur 5 ist nun eine erste mögliche bevorzugte Anbindungsart der Flächendrainage an einen Abflussschlauch dargestellt. In der rechten Hälfte der Fig. 5 ist dabei eine seitliche Ansicht eines Abflussschlauches 3, respektive Zwischenstücks 3', das in gleicher Weise, wie der Abflussschlauch 3 im Übergangsbereich zur Flächendrainage ausführbar ist, dargestellt. Die strichlinierte obere Ausführung in der rechten Abbildung soll dabei lediglich ein ebenfalls mögliches kreisrundes Abflussschlauchende darstellen. In dieses Endstück ist eine schlitzförmige Ausnehmung 33 derart eingebracht, dass in ihr vermittels Kleb- oder sonstiger Verbindung k die Folie 1 und die Stege 2 so aufgenommen werden, dass zumindest die im Beispiel gezeigten äußeren Stege 2 vom Schlauchwandungsmaterial erfasst werden und durch die geschaffene Verbindung zugfest arretiert werden. In weiterer Ausgestaltung dieser Art der Befestigung ist in der Draufsicht (vgl. linke Hälfte der Fig. 5) zur Vergrößerung des Wundsekretzutrittsbereichs eine beidseits dieses Schlauchabschnitts vorgesehen Kehlung 32 am Schlauchende vorgesehen. Diese Kehlung 32 führt zugleich zu einer Verschlankung des Übergangsbereichs, so dass, wenn von vorstehend erwähnter Einführhülse 5 Gebrauch gemacht wird, zusätzlicher Stauraum für die Flachdrainage geschaffen ist. Im unteren Teil von Fig. 5, welche einen Schnitt entlang der Ebene B-B darstellt, ist schematisch gezeigt, wie die äußeren Stege 2 in die Wandung des Abflussschlauches 3 eingebunden und somit zugfest gehaltert sind. In diesem speziellen Beispiel wurde von einer Steg-Folien-Ausbildung nach Fig. 1 Gebrauch gemacht, was andere Steg-Folien-Ausbildungsarten jedoch nicht ausschließt. Diese Ausführung hat den zusätzlichen Vorteil, dass beidseits der Folienflächen anfallendes Wundsekret in den Abflussschlauch geleitet wird.

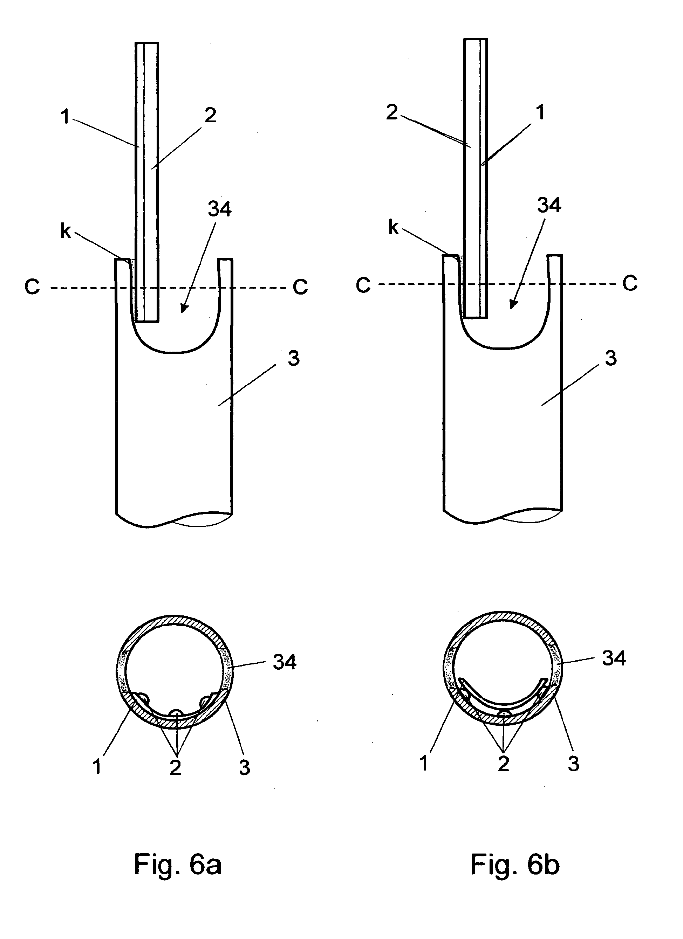

Selbstverständlich liegt es auch im Rahmen der Erfindung, zusätzlich eine U-förmige Abflussschlauchendgestaltung 34, wie in den Figuren 6a und 6b angedeutet, vorzusehen und die korrespondierenden Enden der Stege 2 und/oder der Folie 1 an der Abflussschlauchinnenwand zu befestigen, wie es aus den unteren Schnittbildern entlang einer Ebene C-C ersichtlich ist.

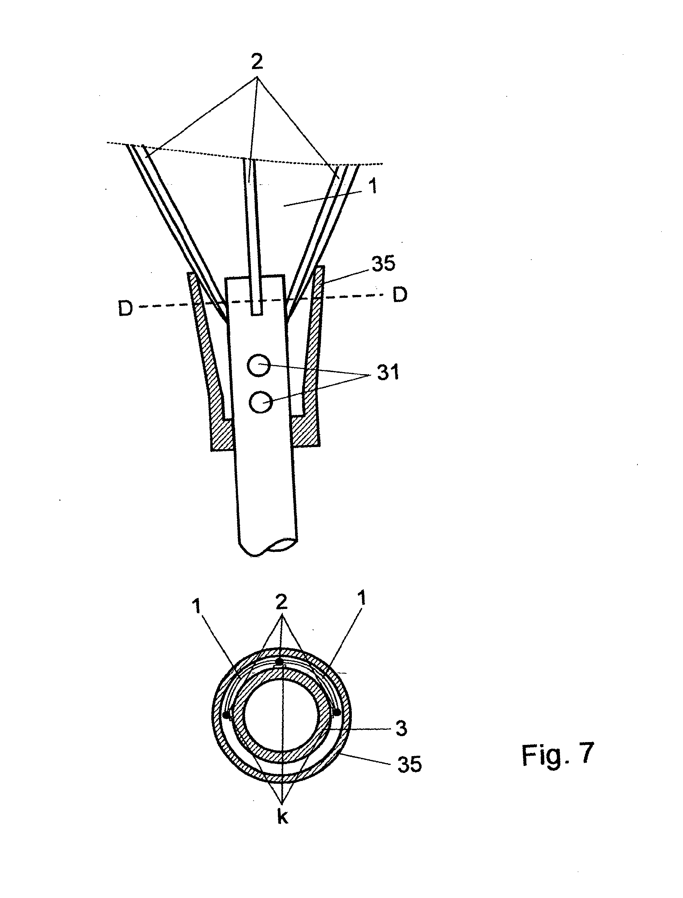

Bei zu erwartendem besonders hohen Anfall an Wundsekretflüssigkeit kann es vorteilhaft sein, auf die eingangs kritisierten Schlauchperforationen 31 nicht gänzlich zu verzichten. Um jedoch deren Tendenz zu Verwachsungen mit dem Gewebe und damit zum ihrem Verschluss zu unterbinden, wird, bei ansonsten, wie vorstehend beschriebener identischer Ausführung der Flächendrainage und deren Anbindung an den Abflussschlauch, im Übergangsbereich zwischen Drainageöffnungen 31 aufweisenden Abflussschlauch 3 und daran angebundener Flächendrainage ein trichterförmig ausgebildeter Einwachsschutz 35 vorgesehen, welcher so ausgebildet ist, das er einen lichten Zwischenraum zu den Perforationen 31 frei hält, außen jedoch eine Beabstandung der Perforationen 31 zum anliegenden Gewebe (hier nicht dargestellt) bildet. Eine solche zusätzliche Ausführung ist in Fig. 7 an einem Beispiel angedeutet. Wird dieser rohr- und trichterförmige Einwachsschutz 35 zusätzlich dichtend aber auf dem Abflussschlauch verschiebbar angeordnet, kann er zusätzlich die von ihm erfassten Stege 2 mit einer Vorspannkraft beaufschlagen. Während im oberen Teil der Fig. 7 eine teilweise geschnittene Draufsicht dargestellt ist, kann dem unteren Teil der Abbildung beispielhaft entnommen werden, wie eine vorteilhafte Anbindung der Stege 2 und der durch sie getragenen Folie 1 in dieser Ausführungsform erfolgt. Hier können die die Folie 1 tragenden Stege 2 am äußeren Umfang des Abflussschlauches 3 und nach außen überdeckt durch den trichterförmigen Einwachsschutz 35 derart angebracht sein, dass auch in diesem Ausführungsbeispiel, bedingt durch die Beabstandung der Folie 1 sowohl zum Einwachsschutz 35 als auch zum Abflussschlauch 3 ein den Wundsekretabfluss ermöglichender Zwischenraum verbleibt. Somit bewirkt auch in diesem Fall die Folie 1 einen Wundsekretabfluss auf beiden ihrer Oberflächen.

Alle vorstehend beschriebenen Anbringungsarten der Stege 2 an den Abflussschlauch lassen sich aber auch besonders elegant durch einen gleichzeitigen Spritzguss der Stege und des Abflussschlauches 3, respektive des Zwischenstücks 3', realisieren, so dass ein einstückiges Spritzstück mit den genannten Baugruppen vorliegt. Da sich die Folie 1 selbst durch dieses Verfahren, nach dem derzeit verfügbaren Stand der Technik, jedoch nicht in der erforderlichen Dünnheit herstellen lässt, wäre diese, in diesem Fall, in einem nachträglichen Arbeitsgang mit den Stegen 2 zu verbinden. Darüber hinaus erlaubt die gemeinsame Herstellungsmethode von Stegen 2 und Abflussschlauch 3, respektive des Zwischenstück 3', die elastischen Stege gleich in der gewünschten Endlage, sprich ihrer gewünschten Ausrichtung im Wundbereich, herzustellen, wodurch die regenschirmartige Entspannung bei der Platzierung der gesamten Flachdrainage unmittelbar gegeben ist. Bei ansonsten gesonderter nachträglicher Anbringung der Stegenden an den Abflussschlauch muss dieser Aspekt bei der Montage gesondert berücksichtigt werden.

Die flächenartige Ausbildung des erfindungsgemäßen Drainagekörpers verhindert wirkungsvoll die Bildung einer Röhre um den platzierten Drain (wie nach dem Stand der Technik üblich) und beugt somit dem Verstopfen der Drainagewege vor. Dadurch wird die Wundheilung verbessert und die Patientensicherheit erhöht.

Durch die hohe Flexibilität der Drainagefläche (Folie) und der Stege kann diese Drainage unkompliziert aus der Wunde entfernt werden, da sie sich selbst bei allen beschriebenen Ausführungen auf den Außendurchmesser des Abflussschlauches zusammenzieht, wenn die Gesamtdrainage aus der Einstichöffnung herausgezogen wird.

Im Rahmen der Erfindung wurde gefunden, dass erfindungsgemäß ausgebildete Flächendrainagen überraschender Weise nicht zu den zu erwarteten Adhäsionen am Körpergewebe führen, sondern einen Sekretfluss über die gesamte angebotene Drainagefläche auch bei längeren Liegezeiten im Wundbereich ermöglichen.

Alle in den Ausführungsbeispielen, den Patentansprüchen und/oder Zeichnungen dargestellten Merkmale können sowohl einzeln als auch in beliebiger Kombination miteinander erfindungswesentlich sein.

### Bezugszeichenliste

- 1: - Folie
- 2: - Steg
- 3: - Abflussschlauch
- 31: - Perforation
- 32: - Kehlung
- 33: - schlitzförmige Ausnehmung
- 34: - U-förmige Ausnehmung
- 35: - Einwachsschutz
- 4: - Pfeile (Sekretfluss)
- 5: - Einführhülse
- 6: - Haut
- 7: - Vakuumquelle
- 8: - Sekretauffangbehälter
- 9: - Geliermasse
- A-A, B-B, C-C, D-D: Schnittebenen
- L: - Folienlänge

## Patentansprüche

1. Flächendrainage zur Ableitung von Wundsekret von großflächigen Wunden und aus Körperhöhlen, umfassend ein Drainagemittel und einen Abflussschlauch (3), **dadurch gekennzeichnet, dass** das Drainagemittel gebildet ist durch wenigstens eine antiadhäsive biokompatible, dünne flexible Folie (1), die von wenigstens zwei, sich über die Folienlänge (L) erstreckende und mit ihr verbundene Stege (2) erfasst ist, wobei zumindest die äußeren Stege (2) in ihrer Geometrie im Wesentlichen der seitlichen Folienberandung folgend festgelegt sind und die Stege (2) einseitig mit dem Abflussschlauch (3), respektive einem Zwischenstück (3'), in eine zugfeste Verbindung gebracht und ausrichtend gehaltert sind und die Folie (1) im Bereich des der Wunde zugewandten Endes des Abflussschlauchs, respektive des Zwischenstücks (3'), in dieses einmündet oder anliegt.

2. Flächendrainage nach Anspruch 1, **dadurch gekennzeichnet, dass** die Stege (2) voneinander beabstandet beidseits von jeweils einer biokompatiblen, dünnen flexiblen Folie (1, 1') erfasst und zwischen diese eingebettet sind.

3. Flächendrainage nach Anspruch 1, **dadurch gekennzeichnet, dass** die Stege (2) voneinander beabstandet in die biokompatible, dünne flexible Folie (1) ein- oder auf diese aufgebracht sind.

4. Flächendrainage nach einem der vorstehenden Ansprüche, **dadurch gekennzeichnet, dass** die Stege (2) voneinander beabstandet durch einen Klebe- oder thermischen Verbindungsprozess mit der biokompatiblen, dünnen flexiblen Folie (1, 1') verbunden sind.

5. Flächendrainage nach einem der vorstehenden Ansprüche, **dadurch gekennzeichnet, dass** die Stege (2) elastisch ausgeführt sind.

6. Flächendrainage nach einem der vorstehenden Ansprüche, **dadurch gekennzeichnet, dass** die Stege (2), mit einer mechanischen Vorspannung beaufschlagt, mit den Abflussschlauch (3), respektive dem Zwischenstück (3'), verbunden sind, wobei die Vorspannkraft ein selbsttätiges Aufspannen der mit ihr verbundenen wenigstens einen Folie (1, 1') bewirkt, sobald die Folie im Wundbereich verlegt ist.

7. Flächendrainage nach einem der vorstehenden Ansprüche, **dadurch gekennzeichnet, dass** die Folie (1, 1') und die sie tragenden Stege (2) vor der Verlegung in den Wundbereich derart zusammenfaltbar oder rollbar sind, dass sie von einer Einführhülse (5) aufgenommen werden können, deren Innendurchmesser den Außendurchmesser des Abflussschlauchs (3), respektive des Zwischenstücks (3'), zu erfassen gestattet.

8. Flächendrainage nach einem der vorstehenden Ansprüche, **dadurch gekennzeichnet, dass** die flexible Folie (1, 1') im aufgespannten Zustand dreieckig, elliptisch, rund oder rechteckig ausgebildet ist.

9. Flächendrainage nach Anspruch 1, **dadurch gekennzeichnet, dass** in das wundseitige Ende des Abflussschlauches (3), respektive des Zwischenstücks (3'), eine schlitzförmige Ausnehmung (33) zur Aufnahme und zugfesten Befestigung von zumindest den äußeren Stegen (2) vorgesehen ist.

10. Flächendrainage nach Anspruch 9, **dadurch gekennzeichnet, dass** das wundseitige Ende des Abflussschlauches (3), respektive des Zwischenstücks (3'), in einer Ebene senkrecht zur vorgesehenen schlitzförmigen Ausnehmung (33) zusätzlich beidseits dieses Schlauchabschnitts mit einer Kehlung (32) versehen ist.

11. Flächendrainage nach Anspruch 1, **dadurch gekennzeichnet, dass** die Stege (2) und/oder die von ihr getragene Folie (1) an der inneren Oberfläche im wundseitigen Endbereich des Abflussschlauches (3), respektive des Zwischenstücks (3'), befestigt sind.

12. Flächendrainage nach Anspruch 1 oder 13, **dadurch gekennzeichnet, dass** das wundseitige Abflussschlauchende zusätzlich mit einer U-förmigen Ausnehmung (34) versehen ist.

13. Flächendrainage nach Anspruch 1 , **dadurch gekennzeichnet, dass** bei einem Abflussschlauches (3), der mit im wundseitigen Bereich liegenden Perforationen (31) versehen ist, diese mit einem trichterförmig ausgebildeten Einwachsschutz (35) derart überdeckt sind, dass ein lichter Zwischenraum zwischen den Perforationen (31) und dem Einwachsschutz (35) verbleibt.

14. Flächendrainage nach Anspruch 13 und einem der vorstehenden Ansprüche, **dadurch gekennzeichnet, dass** die Stege (2) und/oder die von ihr getragene Folie (1) an der äußeren Oberfläche im wundseitigen Endbereich des Abflussschlauches (3), respektive des Zwischenstücks (3'), befestigt sind.

15. Flächendrainage nach einem der vorstehenden Ansprüche, **dadurch gekennzeichnet, dass** die Stege (2) und der Abflussschlauch (3), respektive das Zwischenstück (3'), als einstückiges Teil, vorzugsweise Spritzteil, ausgebildet sind.

## Claims

1. A surface drainage for draining off wound exudation from extensive wounds and body cavities, comprising a drainage means and a drain hose (3), **characterized in that** the drainage means is formed by at least one anti-adhesive biocompatible thin flexible film (1) that is attached to at least two bridges (2) extending over the length (L) of the film and connected thereto, wherein at least the outer bridges (2) in their geometry are defined substantially according to the lateral film boundary, and the bridges (2) on one side are brought into a tension-proof connection with the drain hose (3) or an intermediate piece (3'), respectively, and are fastened in an aligning manner, and the film (1) in the area of the wound-facing end of the drain hose or the intermediate piece (3'), respectively, leads or clings thereto.

2. The surface drainage according to claim 1, **characterized in that** the bridges (2) are encompassed, spaced apart from each other on both sides, each with a biocompatible thin flexible film (1, 1') and are embedded between them.

3. The surface drainage according to claim 1, **characterized in that** the bridges (2) are inserted in or applied to the bio-compatible, thin flexible film (1) in a manner spaced apart from each other.

4. The surface drainage according to any one of the preceding claims, **characterized in that** the bridges (2) are connected to the bio-compatible, thin flexible film (1, 1') by a stick-on or thermal binding process in a manner spaced apart from each other.

5. The surface drainage according to any one of the preceding claims, **characterized in that** the bridges (2) are formed elastically.

6. The surface drainage according to any one of the preceding claims, **characterized in that** the bridges (2), loaded with mechanical pretension, are connected to the drain hose (3) or the intermediate piece (3'), respectively, wherein the tensile force causes an independent stretch out of the at least one film (1, 1') as soon as the film has been put down in the wound area.

7. The surface drainage according to any one of the preceding claims, **characterized in that** the film (1, 1') and the bridges (2) bearing it before put down in the wound area are foldable and rollable such that they can be received by an introduction sleeve (5) the inner diameter of which allows to encompass the outer diameter of the drain hose (3) or intermediate piece (3'), respectively.

8. The surface drainage according to any one of the preceding claims, **characterized in that** the flexible film (1, 1') in the stretched out state is triangular, elliptical, round, or rectangular.

9. The surface drainage according to claim 1, **characterized in that** in the woundside end of the drain hose (3) or the intermediate piece (3'), respectively, there is provided a slit-shaped recess (33) for receiving and tension-proof fastening at least the outer bridges (2).

10. The surface drainage according to claim 9, **characterized in that** the wound-side end of the drain hose (3) or the intermediate piece (3'), respectively, in a plane perpendicular to the provided slit-shaped recess (33) additionally is provided with a gorge (32) at both sides of said hose section.

11. The surface drainage according to claim 1, **characterized in that** the bridges (2), and/or the film (1) carried by them, are attached to the inner surface in the wound-side end area of the drain hose (3) or the intermediate piece (3'), respectively.

12. The surface drainage according to claim 1 or 13, **characterized in that** the wound-side drain hose end additionally is provided with a U-shaped recess (34).

13. The surface drainage according to claim 1, **characterized in that** with a drain hose (3) provided with perforations (31) located in the wound-side area these are covered by a funneled grow-in protection (35) such that there remains a thin interspace between the perforations (31) and the grow-in protection (35).

14. The surface drainage according to claim 13 and any one of the preceding claims, **characterized in that** the bridges (2), and/or the film (1) carried by them, are attached to the outer surface in the wound-side end area of the drain hose (3) or the intermediate piece (3'), respectively.

15. The surface drainage according to any one of the preceding claims, **characterized in that** the bridges (2) and the drain hose (3) or the intermediate piece (3'), respectively, are formed as a one-piece member, preferably an injection-molded article.

## Revendications

1. Drainage de surface pour dévier la sécrétion de plaies de grande surface et hors de cavités corporelles comprenant un moyen de drainage et un tuyau d'écoulement (3), **caractérisé en ce que** le moyen de drainage est formé par au moins une feuille antiadhésive biocompatible fine flexible (1) qui est saisie avec au moins deux traverses (2) s'étendant sur la longueur de la feuille (L) et lui étant associées, au moins les traverses extérieures (2) étant fixées dans une géométrie suivant essentiellement le bord latéral de la feuille et les traverses (2) étant placées d'un côté avec le tuyau d'écoulement (3), respectivement une pièce intermédiaire (3') et maintenues dans une direction dans une association résistant à la traction et la feuille (1) y pénétrant ou s'y posant dans la zone de l'extrémité du tuyau d'écoulement (3), respectivement de la pièce intermédiaire (3') tournée vers la plaie.

2. Drainage de surface selon la revendication 1 **caractérisé en ce que** les traverses (2) sont saisies à une distance l'une de l'autre des deux côtés par respectivement une feuille biocompatible fine flexible (1, 1') et logées entre celle-ci.

3. Drainage de surface selon la revendication 1 **caractérisé en ce que** les traverses (2) sont appliquées à une distance l'une de l'autre dans la feuille biocompatible fine flexible (1) ou sur celle-ci.

4. Drainage de surface selon l'une des revendications précédentes **caractérisé en ce que** les traverses (2) sont associées, à distance l'une de l'autre, par un procédé de raccordement par colle ou thermique à la feuille biocompatible fine flexible (1, 1').

5. Drainage de surface selon l'une des revendications précédentes **caractérisé en ce que** les traverses (2) sont exécutées de façon élastique.

6. Drainage de surface selon l'une des revendications précédentes **caractérisé en ce que** les traverses (2), sollicitées par une précontrainte mécanique, sont associées au tuyau d'écoulement (3), respectivement à la pièce intermédiaire (3'), la force de précontrainte exerçant un étirement automatique d'une moins une feuille (1, 1') associée à cette dernière, dès que la feuille est déplacée dans la zone de la plaie.

7. Drainage de surface selon l'une des revendications précédentes **caractérisé en ce que** la feuille (1, 1') et les traverses (2) la portant peuvent être pliées ou enroulées avant la pose dans la zone de la plaie de façon à ce qu'elles puisent être prises par une douille d'introduction (5) dont le diamètre intérieur permet de saisir le diamètre extérieur du tuyau d'écoulement (3), respectivement de la pièce intermédiaire (3').

8. Drainage de surface selon l'une des revendications précédentes **caractérisé en ce que** la feuille flexible (1, 1') a, une forme triangulaire, elliptique, ronde ou rectangulaire à l'état étiré.

9. Drainage de surface selon la revendication 1 **caractérisé en ce qu'**à l'extrémité côté plaie du tuyau d'écoulement (3), respectivement de la pièce intermédiaire (3') est prévue une cavité en forme de fente (33) pour loger et fixer de façon à résister à la traction des traverses (2) extérieures au moins.

10. Drainage de surface selon la revendication 9 **caractérisé en ce que** l'extrémité côté plaie du tuyau d'écoulement (3), respectivement de la pièce intermédiaire (3') est pourvue en plus des deux côtés de cette section de tuyau d'une cannelure (32) à un niveau verticalement par rapport à la cavité en forme de fente (33) prévue.

11. Drainage de surface selon la revendication 1 **caractérisé en ce que** le traverse (2) et/ou la feuille (1) portée par elle sont fixées à la surface intérieure dans la zone d'extrémité côté plaie du tuyau d'écoulement (3), respectivement de la pièce intermédiaire (3').

12. Drainage de surface selon la revendication 1 ou 13 **caractérisé en ce que** l'extrémité du tuyau d'écoulement côté plaie est pourvue en plus d'une cavité en forme de U (34).

13. Drainage de surface selon la revendication 1 **caractérisé en ce que** pour un tuyau d'écoulement (3) qui est pourvu de perforations (31) se trouvant dans la zone côté plaie, celles-ci sont recouvertes d'une protection adaptable (35) en forme d'entonnoir de façon à ce qu'un espace intermédiaire vide reste entre les perforations (31) et les protection adaptables (35).

14. Drainage de surface selon la revendication 1 et l'une des revendications précédentes **caractérisé en ce que** la traverse (2) et/ou la feuille (1) portée par elle sont fixées à la surface extérieure dans la zone d'extrémité côté plaie du tuyau d'écoulement (3), respectivement de la pièce intermédiaire (3').

15. Drainage de surface selon l'une des revendications précédentes **caractérisé en ce que** la traverse (2) et le tuyau d'écoulement (3), respectivement la pièce intermédiaire (3'), sont formés en une seule pièce, de préférence une pièce moulée par injection.
